# EUROPÄISCHE PATENTSCHRIFT

(11) **EP 2 504 321 B1**
(45) Veröffentlichungstag und Bekanntmachung des Hinweises auf die Patenterteilung: **16.04.2014**
(21) Anmeldenummer: 09759739.7
(22) Anmeldetag: 26.11.2009
(51) Int. Cl.: C07D 261/04

(54) **VERFAHREN ZUR HERSTELLUNG 5,5-DISUBSTITUIERTER 2-ISOXAZOLINE**
METHOD FOR PRODUCING 5,5-DISUBSTITUTED 2-ISOXAZOLINES
PROCÉDÉ DE PRODUCTION DE 2-ISOXAZOLINES 5,5-DISUBSTITUÉES

(43) Veröffentlichungstag der Anmeldung: 03.10.2012
(73) Patentinhaber: BASF SE, 67056 Ludwigshafen (DE)
(72) Erfinder: FRASSETTO, Timo, 68165 Mannheim (DE)
(86) Internationale Anmeldenummer: PCT/EP2009/065927
(87) Internationale Veröffentlichungsnummer: WO 2011/063843

(56) Entgegenhaltungen:
- EP-A1- 1 203 768
- EP-A1- 1 364 946
- WO-A2-2008/000469
- POHJAKALLIO A ET AL: "A versatile Entry to 3-Unsubstituted 2-Isoxazolines" SYNLETT, GEORG THIEME VERLAG, DE LNKD- DOI:10.1055/S-2008-1042900;ART ID:G37807ST, Nr. 6, 11. März 2008 (2008-03-11), Seiten 827-830, XP002526238 ISSN: 0936-5214
- POHJAKALLIO, ANTTI, PIHKO, PETRI M.: "Enantioselective Synthesis of 2-Isoxazolines by a One-Flask Conjugate Addition/Oxime-Transfer Process" CHEM. EUR. J., Bd. 15, 4. März 2009 (2009-03-04), Seiten 3960-3964, XP007914212 DOI: 10.1002/chem.200802684
- GIBERT J-P ET AL: "Action de l'oxyde de benzonitrile sur les isoxazolines-2" BULLETIN DE LA SOCIETE CHIMIQUE DE FRANCE, SOCIETE FRANCAISE DE CHIMIE. PARIS, FRANCE, 1. Januar 1979 (1979-01-01), Seiten 281-288, XP009112841 ISSN: 0037-8968
- ROBERT JACQUIER ET AL: "ACTION L'HYDROXYUREE SUR LES COMPOSES CARBONYLES ETHYLENIQUES. NOUVELLE VOIE DE SYNTHESE D'ISOXAZOLINES-2" TETRAHEDRON LETTERS, ELSEVIER, AMSTERDAM, NL, Nr. 28, 1. Januar 1975 (1975-01-01), Seiten 2337-2340, XP007914198 ISSN: 0040-4039
- ALEXANDER BÖHME, DIANA THAENS, FRANZISKA SCHRAMM, ALBRECHT PASCHKE AND GERRIT SCHÜÜRMANN: "Thiol Reactivity and Its Impact on the Ciliate Toxicity of alpha,beta-unsaturated Aldehydes, Ketones, and Esters", CHEM. RES. TOXICOL, vol. 23, 10 May 2010 (2010-05-10), pages 1905-1912,

## Beschreibung

Die vorliegende Erfindung betrifft ein Verfahren zur Herstellung 5,5-disubstituierter 2-Isoxazoline der Formel (I), 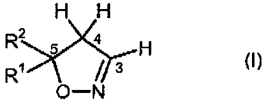 wobei
- R¹, R²: unabhängig voneinander jeweils C₁-C₆-Alkyl oder C₁-C₄-Halogenalkyl bedeuten; oder R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann.

2-Isoxazoline werden typischerweise durch 1,3-dipolare Cycloaddition von Nitriloxiden an Alkene (1) oder die Reaktion α,β-ungesättigter Ketone mit Hydroxylaminen (2) dargestellt (Lang, S. A.; Lin, Y.-I. in Comprehensive Heterocyclic Chemistry, Vol.6, Katritzky, A. R.: Rees, C. W., Eds.; Pergamon: Oxford, 1984, 88-98). Als nachteilig bei Methode (1) hat sich die Tendenz der Nitriloxide zur Dimerisierung erwiesen. Die Synthesestrategie (2) ergibt häufig Produktgemische.

Eine weitere Methode zur Darstellung von 2-Isoxazolinen ist die intramolekulare Umlagerung von O-Propargyl-hydroxylaminen, die jedoch eine aufwändige Synthese der Ausgangsverbindung voraussetzt (Synlett 2006 (3), 463).

Die Cyclisierung von β-Halogen-Ketonoximen setzt ebenfalls den Aufbau des Startmaterials durch mehrstufige Synthese voraus und hat nur vereinzelt Anwendung gefunden (J. Org. Chem. 1970 (35), 2065).

Besonders die Synthese 3-unsubstituierter 2-Isoxazoline nach Methode (1) gestaltet sich schwierig, da die für die Cycloaddition notwendige Knallsäure nur bei tiefen Temperaturen stabil ist und leicht polymerisiert. Die Aktivierung von Nitromethan mit Isocyanaten zur Erzeugung des Nitriloxids gelingt nicht (Chem. Ber. 106 (1973), 3291), und die Aktivierung von Nitromethan mit Trimethylsilylchlorid liefert in den wenigen publizierten Beispielen nur mäßige Ausbeuten (J. Org. Chem. 66 (2001), 2296 und Tetrahedron 39 (1983), 2247).

Die direkte Reaktion α,β-ungesättigter Aldehyde mit Hydroxylaminen zu 2-Isoxazolinen nach Methode (2) ist nicht bekannt. Eine zweistufige Alternative besteht in der Addition von N-Hydroxyhamstoff an α,β-ungesättigte Aldehyde in wässrig-methanolischer Lösung, gefolgt von der sauren Eliminierung von Carbaminsäure zu 2-Isoxazolinen (Tetrahedron Lett. 28 (1975), 2337, Bull. Soc. Chim. 5-6 Pt.2 (1979), 281).

Als einstufige Reaktion ist die Umsetzung α,β-ungesättigter Aldehyde mit Oximen unter Einwirkung einer Säure oder einer Kombination aus Säure und einer stickstoffhaltigen Base zu 5-monosubstituierten 2-Isoxazolinen publiziert (Synlett 2008 (6), 827), jedoch mit geringer Raum-Zeit-Ausbeute und ausschließlich für β-monosubstituierte Aldehyde. Das Scheitern der Addition von Oximen an α-verzweigte, α,β-ungesättigte Aldehyde legt für diese Reaktion die Bedeutung sterischer Faktoren nahe.

Die analoge Michael-Addition von Alkoholen an α,β-ungesättigte Aldehyde unter Organokatalyse zeigt eine starke Abhängigkeit der Ausbeute vom sterischen Anspruch des Substrats in β-Position, wobei große Substituenten die niedrigsten Ausbeuten ergeben (Tetrahedron Let. 47 (2006) 3039-3041).
Auch die säurekatalysierte Addition von Alkoholen an α,β-ungesättigte Ketone ergibt nur im Falle terminaler oder β-monosubstituierter Enone befriedigende Ausbeuten. (Org. Lett., Vol.5, No. 12, 2141-44).

Die Herstellung von 2-Isoxazolinen wird weiterhin in Pohjakallio et al. (Chem. Eur. J 2009 (15), 3960), EP 1 203 768 A1, EP 1 364 946 A1 und WO2008/000469 beschrieben.

5,5-disubstituierte 2-Isoxazoline der Formel (I) sind wichtige Zwischenprodukte für die Herstellung agrochemischer und pharmazeutischer Wirkstoffe.

Aufgabe der vorliegenden Erfindung war demzufolge die Bereitstellung eines kostengünstigen, wirtschaftlichen und sicheren, für die großtechnische Anwendung geeigneten Verfahrens zur Herstellung 5,5-disubstituierter 2-Isoxazoline der Formel (I).

Überraschend wurde gefunden, dass 5,5-disubstituierte 2-Isoxazoline der Formel (I) 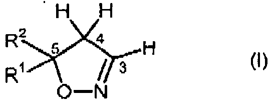 in einem einstufigen Verfahren in sehr guter Ausbeute aus β-disubstituierten Aldehyden ethalten werden, wobei
- R¹, R²: unabhängig voneinander jeweils C₁-C₆-Alkyl oder C₁-C₄-Halogenalkyl bedeuten; oder R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann;
dadurch gekennzeichnet, dass ein Oxim der Formel (II) 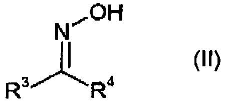 wobei
- R³, R⁴: unabhängig voneinander jeweils Wasserstoff, C₁-C₆-Alkyl, C₁-C₄-Alkylcarbonyl, Hydroxyimino-C₁-C₄-alkyl, Phenyl, Phenyl-C₁-C₄-Alkyl oder Phenyl-C₂-C₄-Alkenyl bedeuten, wobei die Phenylringe ein- oder mehrfach durch C₁-C₄-Alkyl, C₁-C₄-Alkoxy, Di-C₁-C₄-Alkylamino, Halogen, Hydroxy oder Nitro substituiert sein können; oder R³ und R⁴ zusammen eine C₂-C₅-Alkandiyl-Kette bilden.
mit einer Carbonylverbindung der Formel (III), 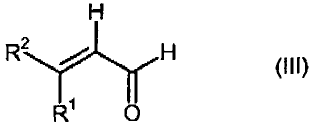 wobei R¹ und R² die oben genannten Bedeutungen haben;
in Anwesenheit eines Säurekatalysators oder eines Säure-Base-Katalysators und gegebenenfalls in Anwesenheit eines organischen Lösungsmittels umgesetzt wird.

Gegenstand der vorliegenden Anmeldung ist daher das erfindungsgemäße Verfahren zur Herstellung 5,5-disubstituierter 2-Isoxazoline der Formel (I).

5,5-disubstituierte 2-Isoxazoline sind Zwischenprodukte in einem Verfahren zur Herstellung von Oxazol-Herbiziden der Formel (IV), 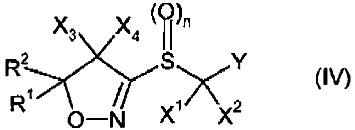 wobei die Variablen folgende Bedeutungen haben:
- n: 0, 1 oder 2;
- X¹, X², X³, X⁴: jeweils unabhängig voneinander Wasserstoff, Fluor oder Chlor; und
- Y: Phenyl, 6-gliedriges Heteroaryl mit ein bis drei Stickstoffatomen oder 5-gliedriges Heteroaryl mit ein bis drei Heteroatomen ausgewählt aus der Gruppe Sauerstoff, Stickstoff und Schwefel, wobei Phenyl und Heteroaryl jeweils durch ein bis fünf Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, Sulfonyl-C₁-C₄-Alkyl, C₂-C₆-Alkenyl, C₂-C₈-Alkinyl, C₁-C₄-Alkoxy, C₁-C₄-Haloalkoxy, C₂-C₆-Alkenyloxy, C₂-C₆-Alkinyloxy und C₁-C₄-Alkylcarbonyloxy, substituiert sein können; und
- R¹, R²: unabhängig voneinander jeweils C₁-C₆-Alkyl oder C₁-C₄-Halogenalkyl; oder R¹ und R² bilden zusammen eine C₂-C₅-Alkandiyl-Kette, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann;

Oxazol-Herbizide der Formel (IV) sind aus WO 02/062770 und WO 01/012613 bekannt.

Weitere Ausführungsformen der vorliegenden Erfindung sind den Ansprüchen, der Beschreibung und den Beispielen zu entnehmen. Es versteht sich, dass die vorstehend genannten und die nachstehend noch zu erläuternden Merkmale des erfindungsgemäßen Gegenstandes nicht nur in der jeweils angegebenen Kombination, sondern auch in anderen Kombinationen verwendbar sind, ohne den Rahmen der Erfindung zu verlassen.

Die für die Substituenten genannten organischen Molekülteile stellen Sammelbegriffe für individuelle Aufzählungen der einzelnen Gruppenmitglieder dar. Kohlenwasserstoffketten können geradkettig oder verzweigt sein. Sofern nicht anders angegeben tragen halogenierte Substituenten vorzugsweise ein bis fünf gleiche oder verschiedene Halogenatome.

Die Bedeutung Halogen steht jeweils für Fluor, Chlor, Brom oder Iod, vorzugsweise Fluor oder Chlor.

Ferner bedeuten beispielsweise:
Alkyl beziehungsweise die Alkylteile von Carboxyalkyl, Sulfonylalkyl, Phenylalkyl, Aryl-alkyl, Heterocyclylalkyl, Heteroarylalkyl, Alkoxy, Alkylcarbonyl, Hydoxyiminoalkyl, Alkylamino, Alkylcarbonyloxy, Alkylsilyl und Alkylsilyloxy bedeuten eine gesättigte, geradkettige oder verzweigte Kohlenwasserstoffgruppe mit 1 bis 6 beziehungsweise 1 bis 4 Kohlenstoffatomen, beispielsweise Methyl, Ethyl, Propyl, 1-Methylethyl, Butyl, 1-Methylpropyl, 2-Methylpropyl, 1,1-Dimethyl-ethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl und deren Isomere. C₁-C₄-Alkyl umfasst beispielsweise Methyl, Ethyl, Propyl, 1-Methylethyl, Butyl, 1-Methylpropyl, 2-Methylpropyl oder 1,1-Dimethyl-ethyl.

Cycloalkyl bezeichnet monocyclische, gesättigte Kohlenwasserstoffgruppen mit drei oder mehr C-Atomen, beispielsweise 3 bis 6 Kohlenstoffringgliedern, wie Cyclopropyl, Cyclobutyl, Cyclopentyl und Cyclohexyl.

Halogenalkyl beziehungsweise die Halogenalkylteile von Haloalkoxy stehen für teilweise oder vollständig halogeniertes Alkyl, wobei das/die Halogenatom(e) insbesondere Fluor, Chlor und/oder Brom ist/sind, beispielsweise Chlormethyl, Brommethyl, Dichlormethyl, Trichlormethyl, Fluormethyl, Difluormethyl, Trifluormethyl, Chlorfluormethyl, Dichlorfluormethyl, Chlordifluormethyl, 1-Chlorethyl, 1-Bromethyl, 1-Fluorethyl, 2-Fluorethyl, 2,2-Difluorethyl, 2-Chlor-2-fluor-ethyl, 2,2,2-Trifluorethyl, 2-Chlor-1,1,2-trifluorethyl, 2-Chlor-2,2-difluorethyl, 2-Brom-2,2-difluorethyl, 2,2-Dichlor-2-fluorethyl, 2,2,2-Trichlorethyl, 1,1,2,2-Tetrafluorethyl, 1,1,2,2-Tetrachlorethyl, Pentafluorethyl, 2,2,3,3-Tetrafluor-1-propyl, 1,1,2,3,3,3-Hexafluor-1-propyl, 1,1,1,3,3,3-Hexafluor-2-propyl, Heptafluor-1-propyl, Heptafluor-2-propyl, 2,2,3,3,4,4,4-Heptafluor-1-butyl oder Nonafluor-1-butyl.

Alkenyl beziehungsweise die Alkenylteile von Phenylalkenyl und Alkenyloxy bedeuten eine einfach ungesättigte, geradkettige oder verzweigte Kohlenwasserstoffgruppe mit zwei bis sechs beziehungsweise zwei bis vier Kohlenstoffatomen und einer Doppelbin-dung in einer beliebigen Position, beispielsweise Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Dimethyl-3-butenyl, 1,2-Dimethyl-1-butenyl, 1,2-Dimethyl-2-butenyl, 1,2-Dimethyl-3-buteriyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, 1-Ethyl-2-methyl-1-propenyl, 1-Ethyl-2-methyl-2-propenyl.

Alkinyl beziehungsweise die Alkinylteile von Alkinyloxy bezeichnen geradkettige oder verzweigte Kohlenwasserstoffgruppen mit zwei oder mehr C-Atomen, beispielsweise 2 bis 4, 2 bis 6, oder 3 bis 6 Kohlenstoffatomen und einer Dreifachbindung, wie Ethinyl, 1-Propinyl, 2-Propinyl, 1-Butinyl, 2-Butinyl, 3-Butinyl, 1-Methyl-2-propinyl, 1-Pentinyl, 2-Pentinyl, 3-Pentinyl, 4-Pentinyl, 1-Methyl-2-butinyl, 1-Methyl-3-butinyl, 2-Methyl-3-butinyl, 3-Methyl-1-butinyl, 1,1-Dimethyl-2-propinyl, 1-Ethyl-2-propinyl, 1-Hexinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-1-pentinyl, 3-Methyl-4-pentinyl, 4-Methyl-1-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 3,3-Dimethyl-1-butinyl, 1-Ethyl-2-butinyl, 1-Ethyl-3-butinyl, 2-Ethyl-3-butinyl, 1-Ethyl-1-methyl-2-propinyl.

Aryl bezeichnet einen ein- bis dreikerniger aromatischer Carbocyclus mit 6 bis 14 Ringgliedern, beispielsweise Phenyl, Naphthyl und Anthracenyl.

Heteroaryl bezeichnet ein 5- oder 6-gliedriges aromatisches Ringsystem mit ein bis vier Stickstoffatomen oder mit ein bis drei Stickstoffatomen und einem Sauerstoff- oder Schwefelatom, oder mit einem Sauerstoff- oder Schwefelatom.

Heterocyclyl bezeichnet einen gesättigten, partiell ungesättigten oder aromatischen heterocyclischer Ring mit drei oder mehr C-Atomen, z.B. 3-, 4-. 5- oder 6-gliedriger heterocyclischer Ring, der ein bis vier gleiche oder verschiedene Heteroatome, ausgewählt aus der Gruppe Sauerstoff, Schwefel und Stickstoff enthält, und über C oder N gebunden sein kann; wobei ein Schwefel im Heterocyclyl zu S=O oder S(=O)₂ oxidiert sein kann, und wobei mit einem ankondensierten Phenylring oder mit einem C₃-C₆-Carbocyclus oder mit einem weiteren 5- bis 6-gliedrigen Heterocyclus ein bicyclisches Ringsystem ausgebildet werden kann.

Im erfindungsgemäßen Verfahren werden bevorzugt Verbindungen der Formeln (II) und (III) eingesetzt, wobei die Variablen jeweils für sich allein oder in Kombination folgende Bedeutungen haben:
- R¹: C₁-C₆-Alkyl oder C₁-C₄-Halogenalkyl;
- R²: C₁-C₆-Alkyl oder C₁-C₄-Halogenalkyl;
- R³: Wasserstoff, C₁-C₆-Alkyl, C₁-C₄-Alkylcarbonyl, Hydroxyimino-C₁-C₄-alkyl; und
- R⁴: C₁-C₆-Alkyl; oder R³ und R⁴ eine C₂-C₅-Alkandiyl-Kette bilden.

Besonders bevorzugt werden Verbindungen der Formeln (II) und (III) eingesetzt, wobei die Variablen jeweils für sich allein oder in Kombination folgende Bedeutungen haben:
- R¹: C₁-C₄-Alkyl, insbesondere Methyl oder Ethyl, besonders bevorzugt Methyl;
- R²: C₁-C₄-Alkyl, insbesondere Methyl oder Ethyl, besonders bevorzugt Methyl;
- R³: Wasserstoff, C₁-C₄-Alkyl, insbesondere Methyl, Ethyl, Isopropyl oder Isobutyl, besonders bevorzugt Methyl und Ethyl; und
- R⁴: C₁-C₄-Alkyl, insbesondere Methyl oder Ethyl; oder R³ und R⁴ eine C₂-C₅-Alkandiyl-Kette bilden.

Außerordentlich bevorzugt werden Verbindungen der Formeln (II) und (III) eingesetzt, wobei die Variablen jeweils für sich allein oder in Kombination folgende Bedeutungen haben:
- R¹: Methyl;
- R²: Methyl;
- R³: Methyl oder Ethyl;
- R⁴: Methyl oder Ethyl.

Eine besonders bevorzugtes 5,5-disubstituiertes 2-Isoxazolin der Formel (I), dass nach dem erfindungsgemäßen Verfahren hergestellt wird, ist 5,5-Dimethyl-2-Isoxazolin (Ia), wobei R¹ und R² in Formel (I) jeweils Methyl bedeuten.

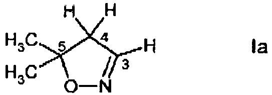

Das 5,5-disubstituierte 2-Isoxazolin der Formel (Ia) mit R¹ und R² in der Bedeutung Methyl wird bevorzugt als Zwischenprodukt in Verfahren zur Herstellung von Oxazol-Herbiziden der Formel (IVB) verwendet, wobei 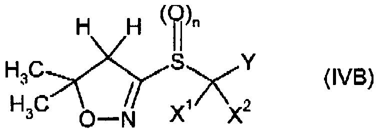
- n: 1 oder 2;
- X¹, X²: jeweils unabhängig voneinander Wasserstoff oder Fluor; und
- Y: Phenyl bedeuten, wobei Phenyl durch ein bis drei Substituenten ausgewählt aus der Gruppe Halogen, C₁-C₄-Alkyl, C₁-C₄-Halogenalkyl und C₁-C₄-Alkoxy substituiert sein kann.

Insbesondere wird das 5,5-disubstituierte 2-Isoxazolin der Formel (Ia) mit R¹ und R² in der Bedeutung Methyl als Zwischenprodukt in Verfahren zur Herstellung auch von Oxazol-Herbiziden der Formel (IVA) verwendet, wobei 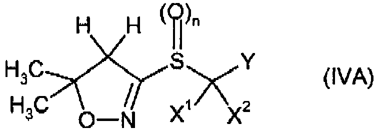
- n: 1 oder 2;
- X¹, X²: jeweils unabhängig voneinander Wasserstoff oder Fluor; und
- Y: Pyrazolyl bedeuten, welches durch ein bis drei Substituenten ausgewählt aus der Gruppe C₁-C₄-Alkyl, C₁-C₄-Halogenalkyl und C₁-C₄-Halogenalkoxy substituiert sein kann.

Außerordentlich bevorzugt wird das 5,5-disubstituierte 2-Isoxazolin der Formel (Ia) als Zwischenprodukt in Verfahren zur Herstellung der Oxazol-Herbizide der Formeln (IVa) und (IVb) verwendet.

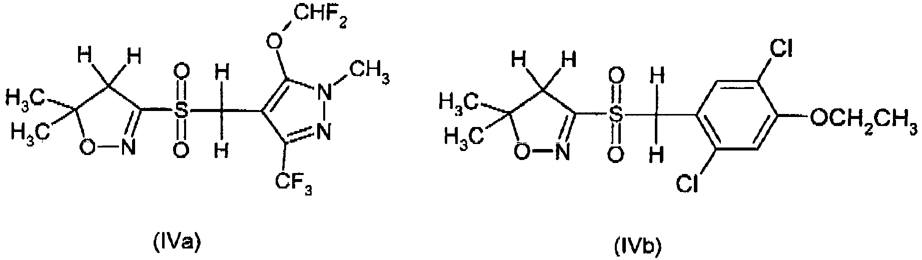

Ausgehend von Zwischenprodukten der Formel (I) sind die weiteren Schritte im Verfahren zur Herstellung von Oxazol-Herbiziden der Formel (IV) dem Fachmann an sich bereits bekannt oder wurden in der Literatur beschrieben.
Für die Verbindung der Formel (IVa) lässt sich eine mögliche Synthese wie folgt darstellen:

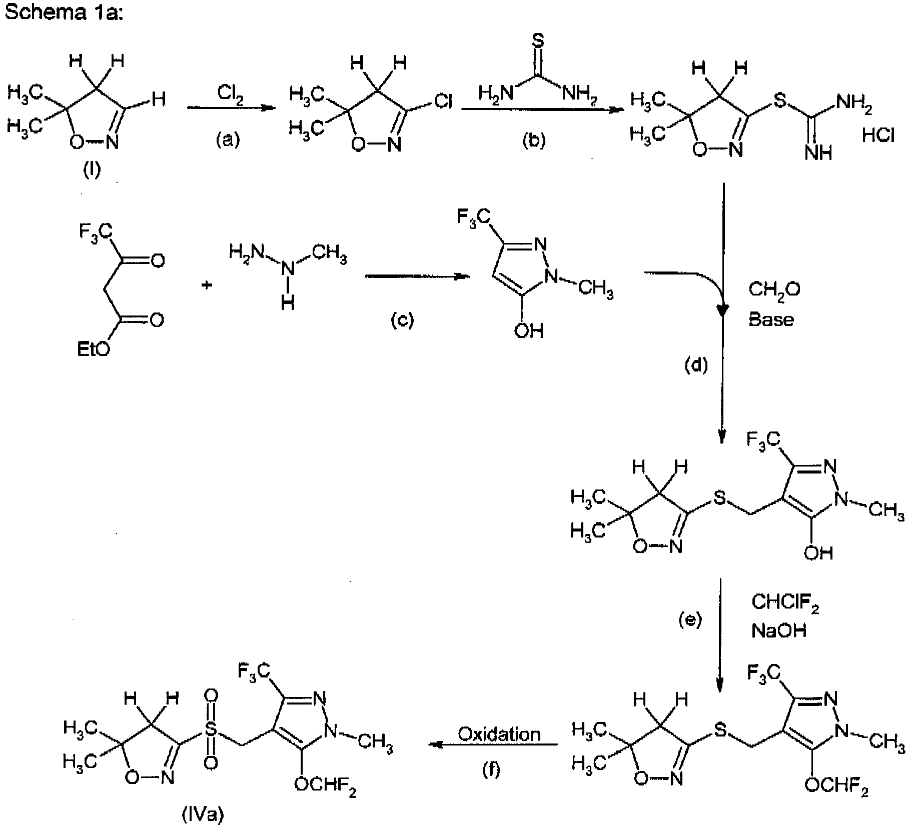

Die Chlorierung (a) 3-unsubstituierter Isoxazoline mit Chlorgas ist dem Fachmann bekannt (J. Org. Chem. 53 (1988), 4074-81). Die nachfolgende Umsetzung mit Thioharnstoff zu Thiocarboxamidin-Salzen (b) wird in EP 1 829 868 beschrieben. Die Darstellung des Pyrazols (c) aus Dicarbonylverbindungen und Hydrazinen wird beispielsweise in JP2007/031343 demonstriert. Die Umsetzung des Hydroxy-substituierten Pyrazols mit Formaldehyd, gefolgt von der Reaktion mit Thiocarboxamidin-Salzen (d) wird in CA 2 560 936 (WO2005/095352) beschrieben. Die Alkylierung der Hydroxygruppe (e) ist dem Fachmann aus JP2007/246396 bekannt. Die abschließende Oxidation des Schwefels zum Sulfon (f) wird beispielsweise in EP 1 405 853 angewandt.

Für die Verbindung der Formel (IVb) lässt sich eine mögliche Synthese wie folgt darstellen:

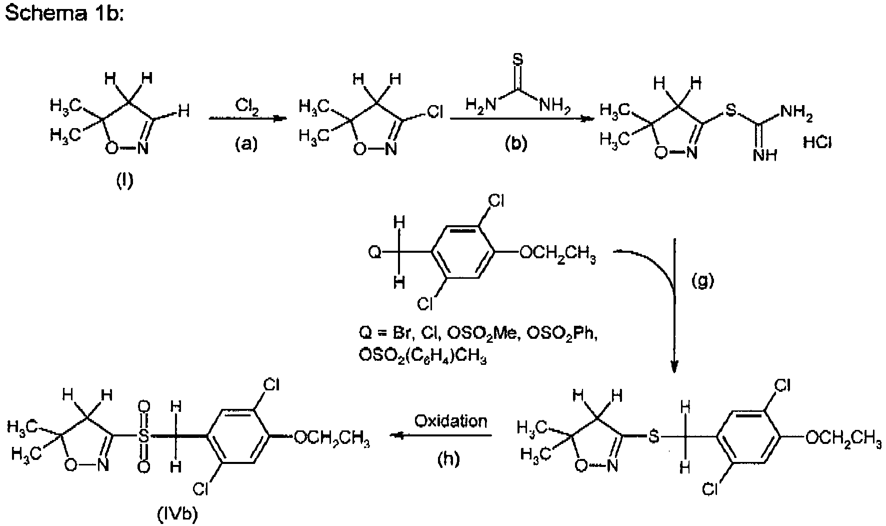

Die Chlorierung (a) 3-unsubstituierter Isoxazoline mit Chlorgas ist dem Fachmann bekannt (J. Org. Chem. 53 (1988), 4074-81). Die nachfolgende Umsetzung mit Thiohamstoff zu Thiocarboxamidin-Salzen (b) wird in EP 1 829 868 beschrieben. Die nucleophile Substitution benzylischer Bromide (g) wurde beilspielsweise in WO2007/096576 demonstriert. Die abschließende Oxidation des Schwefels zum Sulfon (h) wird beispielsweise in EP 1 405 853 angewandt.

Im erfindungsgemäßen Verfahren wird das Oxim der Formel (II) mit der Carbonylverbindung der Formel (III) in Anwesenheit eines Säurekatalysators oder eines Säure-Base-Katalysators und gegebenenfalls in Anwesenheit eines organischen Lösungsmittels umgesetzt (Schema 2):

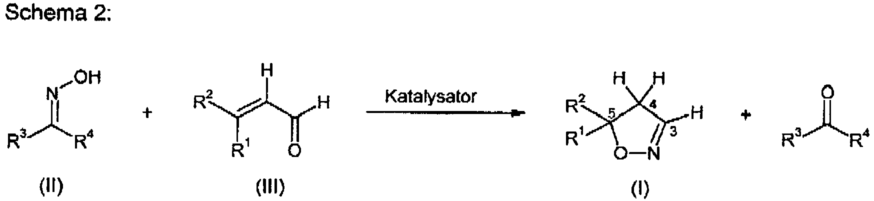

Die Oxime der Formel (II) sind entweder kommerziell erhältlich oder können beispielsweise gemäß Yamane, M.; Narasaka, K., in Science of Synthesis, 27 (2004), S.605 hergestellt werden. Die Carbonylverbindungen der Formel (III) sind ebenfalls kommerziell erhältlich oder können beispielsweise gemäß Escher, I.; Glorius, F., in Science of Synthesis, 25 (2006), S. 733 hergestellt werden.

In der Regel wird das erfindungsgemäße Verfahren zur Herstellung der Verbindung der Formel (I) unter folgenden Bedingungen durchgeführt:
Das Oxim der Formel (II) und die Carbonylverbindung der Formel (III) werden im erfindungsgemäßen Verfahren in einem Molverhältnis von 3:1 bis 1:3 eingesetzt. Bevorzugt beträgt der Überschuss einer der beiden Komponenten bis zu 20 mol%, insbesondere der Carbonylverbindung der Formel (III). Das bevorzugte Molverhältnis des Oxims (II) zur Carbonylverbindung (III) liegt entsprechend bei 1.0:0.8 bis 1.0:1.2, besonders bevorzugt bei etwa 1.0:1.0 bis 1.0:1.1.

Die Umsetzung des Oxims der Formel (II) und der Carbonylverbindung der Formel (III) findet in Gegenwart eines Katalysators statt. Geeignete Katalysatoren sind bestimmte Säuren (Säurekatalysator) oder Mischungen bestimmter Säuren und bestimmter Basen (Säure-Base-Katalysator).

Das erfindungsgemäße säurekatalysierte Verfahren kann schematisch wie folgt dargestellt werden:

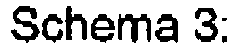

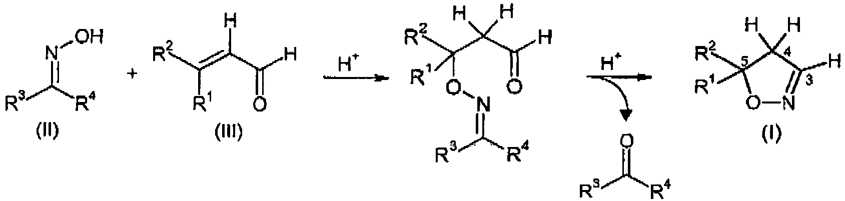

Geeignete Säurekatalysatoren sind Protonendonatoren (Brönstedt-Säuren), beispielsweise anorganische und organische Säuren. Beispiele für anorganische Säuren sind Halogenwasserstoffsäuren und Sauerstoffsäuren, insbesondere Salzsäure, Schwefelsäure, Salpetersäure, Phosphorsäure, Phosphonsäure und Phosphinsäure.

Beispiele für organische Säuren sind aliphatische und aromatische Säuren wie Alkylsulfonsäuren, Arylsulfonsäuren, Mono-C₁-C₆-alkylphosphate, Di-C₁-C₆-alkylphosphate, Monoarylphosphate, Diarylphosphate, Alkylcarbonsäuren, Halogenalkylcarbonsäuren und Heterocyclylcarbonsäuren, insbesondere Methansulfonsäure, p-Toluolsulfonsäure, Zitronensäure, Trifluoressigsäure und Prolin.

Im allgemeinen läuft die Reaktion säurekatalysiert mit solchen Säuren in guter Ausbeute ab, deren pKs-Wert kleiner als 3.5 ist.

Wird das erfindungsgemäße Verfahren nur unter Säurekatalyse durchgeführt, so werden bevorzugt starke Säuren wie Phosphorsäure, Mono-C₁-C₆-alkylphosphate, Di-C₁-C₆-alkylphosphate, Monoarylphosphate, Diarylphosphate, Schwefelsäure, Sulfonsäuren oder Trifluoressigsäure verwendet.

Das erfindungsgemäße säure-base-katalysierte Verfahren könnte nach folgendem Schema ablaufen:

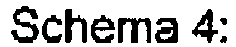

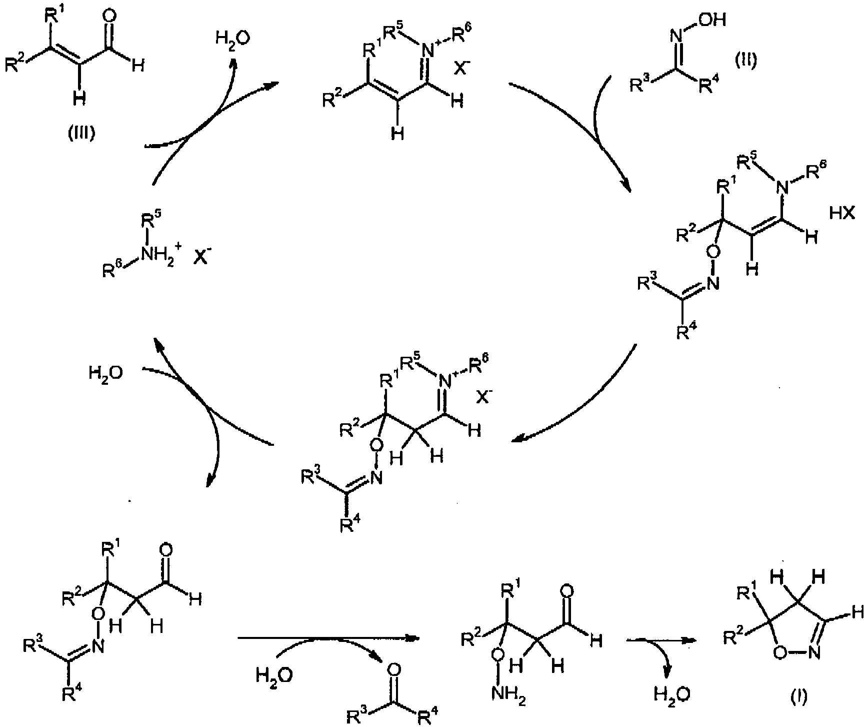

Geeignete Säure-Base-Katalysatoren sind Mischungen aus den oben beschriebenen Säuren und bestimmten Basen, wobei Säure und Base getrennt voneinander oder als Säureadditionssalz eingesetzt werden können.

Als geeignete Basen haben sich Verbindungen erwiesen, die ein oder mehrere Heteroatome enthalten, beispielsweise Stickstoff, Sauerstoff, Schwefel oder Phosphor, wobei Stickstoff ein bevorzugtes Heteroatom ist.

Beispiele für solche Basen sind primäre oder sekundäre Amine der Formel (V) 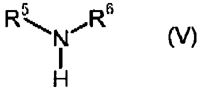 wobei R⁵ und R⁶ unabhängig voneinander jeweils C₁-C₆-Alkyl, C₃-C₆-Cycloalkyl, C₂-C₆-Alkenyl, C₂-C₆-Alkinyl, Tri-C₁-C₆-Alkylsilyl, Aryl, Aryl-C₁-C₆-Alkyl, Heteroaryl, Heteroaryl-C₁-C₆-Alkyl bedeuten, und wobei die Aryl und Heteroarylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy, C₁-C₄-Alkylcarbonyloxy substituiert sein können;
und wobei R⁵ zusätzlich Wasserstoff bedeuten kann.

Bevorzugt sind Verbindungen der Formel (V), in denen R⁵ und R⁶ unabhängig voneinander jeweils C₁-C₆-Alkyl, C₃-C₆-Cycloalkyl, Tri-C₁-C₆-Alkylsilyl, Aryl oder Aryl-C₁-C₆-Alkyl bedeuten; und wobei die Arylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy, C₁-C₄-Alkylcarbonyloxy substituiert sein können;
und wobei R⁵ zusätzlich Wasserstoff bedeuten kann.

Besonders bevorzugt sind Verbindungen der Formel (V), in denen R⁵ und R⁶ unabhängig voneinander jeweils Methyl, Ethyl, Propyl, 1-Methylethyl, Butyl, Cyclopentyl, Cyclohexyl, Phenyl, Naphthyl, Benzyl oder Trimethylsilyl bedeuten; und wobei R⁵ zusätzlich Wasserstoff bedeuten kann; beispielsweise N-Methylanilin.

Alternativ können R⁵ und R⁶ gemeinsam eine Ringstruktur der Formel (VI) bilden, 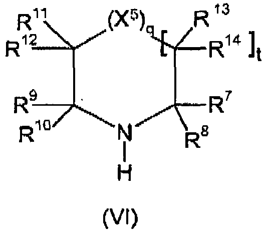 wobei
X⁵ für O, S, NR¹⁵ oder CR¹⁶R¹⁷ steht;
q 0 oder 1 ist;
t 0 oder 1 ist; und
R⁷, R⁸, R⁹, R¹⁰, R¹¹, R¹², R¹³, R¹⁴, R¹⁶, R¹⁷ unabhängig voneinander ausgewählt sind aus Wasserstoff, Hydroxy, Carboxy, Amino, Nitro, Aminocarbonyl, C₁-C₆-Alkyl, C₁-C₆-Alkoxy, C₁-C₆-Alkylcarbonyl, Mono-C₁-C₆-alkylamino, Di-C₁-C₆-alkylamino, Aryl, Heteroaryl, Aryl-C₁-C₆-alkoxy, und wobei C₁-C₆-Alkyl selbst durch Aryl, Heteroaryl, Heterocyclyl oder Trimethylsilyloxy substituiert sein kann und wobei die Aryl, Heterocyclyl und Heteroarylteile der Substituenten selbst durch ein bis drei Substituenten ausgewählt aus der Gruppe Halogen, Nitro, Cyano, C₁-C₄-Alkyl, C₃-C₆-Cycloalkyl, C₁-C₄-Halogenalkyl, Carboxy-C₁-C₄-Alkyl, C₂-C₆-Alkenyl, C₂-C₆-Alkinyl, C₁-C₄-Alkoxy, C₁-C₄-Haloalkoxy, C₂-C₆-Alkenyloxy, C₂-C₆-Alkinyloxy und C₁-C₄-Alkylcarbonyloxy substituiert sein können;
oder R¹¹ und R¹² und/oder R¹³ und R¹⁴ bilden gemeinsam mit dem Kohlenstoffatom, an das sie gebunden sind, eine Ketogruppe; und R¹⁵ ist ausgewählt aus Wasserstoff, C₁-C₆-Alkyl, Aryl-C₁-C₆-alkyl.

Bevorzugt sind Amine der Formel (V) beziehungsweise (VI), wobei R⁵ und R⁶ gemeinsam mit der NH-Gruppe einen Imidazolin-5-on Ring der Formel (VII) bilden, 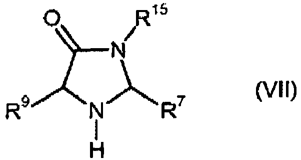 wobei die Substituenten die oben genannte Definition haben.

Besonders bevorzugt sind hierbei Verbindungen, bei denen R⁷ und R⁹ unabhängig voneinander ausgewählt sind aus C₁-C₆-Alkyl und Aryl-C₁-C₆-alkyl, vorzugsweise aus Methyl, Ethyl, 1-Methylethyl, 1,1-Dimethylethyl und Phenylmethyl.

In der Regel werden die Katalysatoren der Reaktionsmischung In katalytischer Menge zugesetzt. In einer erfindungsgemäßen Ausführungsform liegt das Molverhältnis der Verbindung der Formel II zu den Säurekatalysatoren beziehungsweise zu den Säure-Base-Katalysatoren unter 1:0.1. In einer bevorzugten Ausführungsform liegt das Molverhältnis unter 1:0.05, in einer besonders bevorzugten Ausführungsform unter 1:0.02.

Das Oxim der Formel (II) und die Carbonylverbindung der Formel (III) können erfindungsgemäß sowohl ohne Zugabe eines Lösungsmittels als auch unter Zugabe eines geeigneten Lösungsmittels umgesetzt werden.

Gemäß einer Ausführungsform des erfindungsgemäßen Verfahrens werden das Oxim der Formel (II) und die Carbonylverbindung der Formel (III) unter Zugabe eines Lösungsmittels miteinander umgesetzt

Geeignete Lösungsmittel sind organische Lösungsmittel, beispielsweise aromatische Kohlenwasserstoffe wie Toluol, o-, m-, p-Dimethylbenzol, 1,3,5-Trimethylbenzol, Ethylbenzol, Chlorbenzol, o-,m-,p-Dichlorbenzol, halogenierte aliphatische Kohlenwasserstoffe wie Tetrachlorethan, Trichlormethan, Dichlormethan und Dichlorethen, aliphatische Kohlenwasserstoffe wie Pentan, Hexan, Heptan, Octan, Cyclopentan, Methylcyclopentan und Cyclohexan, Ether wie Diethylether, Methyl-tert.-butylether, Tetrahydrofuran und Dioxan, Alkohole wie Methanol und Ethanol, Ester wie Essigsäureethylester, Nitrite wie Acetonitril oder Mischungen der genannten Lösungsmittel.

Bevorzugte Lösungsmittel sind aromatische Kohlenwasserstoffe, halogenierte aliphatische Kohlenwasserstoffe, aliphatische Kohlenwasserstoffe, Ether und Alkohole.

Besonders bevorzugte Lösungsmittel sind aromatische Kohlenwasserstoffe, insbesondere Toluol, Chlorbenzol, Dichlorbenzol und Mischungen dieser Lösungsmittel.

In einer bevorzugten Ausführungsform des erfindungsgemäßen Verfahrens beträgt der Anteil des Lösungsmittels im Reaktionsansatz, also vor Reaktionsbeginn, weniger als 80 Gew.%. In einer besonders bevorzugten Ausführungsform beträgt der Anteil des Lösungsmittels weniger als 50 Gew.%, ganz besonders bevorzugt weniger als 10 Gew.%.

Gemäß einer weiteren besonders bevorzugten Ausführungsform des erfindungsgemäßen Verfahrens werden das Oxim der Formel (II) und die Carbonylverbindung der Formel (III) ohne Zugabe eines Lösungsmittels miteinander umgesetzt.

In der Regel kommt es nicht darauf an, in welcher Reihenfolge das Oxim der Formel (II), die Carbonylverbindung der Formel (III), der Katalysator und gegebenenfalls das Lösungsmittel in das Reaktionsgefäß vorgelegt beziehungsweise zugegeben werden. Gemäß einer Ausführungsform der Erfindung werden das Oxim der Formel (II) und die Carbonylverbindung der Formel (III) und gegebenenfalls das Lösungsmittel vorgelegt und die gewünschte Temperatur eingestellt. Anschließend wird der Katalysator zugegeben.
Gemäß einer anderen Ausführungsform der Erfindung werden das Oxim der Formel (II), der Katalysator und gegebenenfalls das Lösungsmittel vorgelegt und die gewünschte Temperatur eingestellt. Anschließend wird die Carbonylverbindung der Formel (III) zugegeben.

Unter Zugabe versteht man dabei sowohl die portionsweise als auch kontinuierliche Zugabe eines Stoffes. Die Zugabe des Katalysators beziehungsweise der Carbonylverbindung der Formel (III) erfolgt vorzugsweise ohne Lösungsmittel oder gelöst in einem wie oben definierten organischen Lösungsmittel im Verlauf der Reaktion. Normalerweise arbeitet man bei einer Reaktionstemperatur von -40 bis 100°C, vorzugsweise von -20 bis 60°C, insbesondere von 0 bis 30°C.
Das Reaktionsgemisch kann ohne weitere Aufarbeitung direkt anderen Verfahren zugeführt werden. Das Reaktionsprodukt, das 5,5-disubstituierte 2-Isoxazolin der Formel (I), kann auch aus dem Reaktionsgemisch abgetrennt werden, beispielsweise durch direkte Destillation, Extraktion oder Chromatographie, bevorzugt durch Destillation.

### Ausführungsbeispiele:

### Beispiel 1: (Säure-Base Katalyse)

### Synthese von 5,5-Dimethyl-2-isoxazolin

10,0 g (0,137 mol; 100 mol%) Acetonoxim wurden mit 12,7 g (0,151 mol, 110 mol%) 3-Methyl-2-butenal versetzt und auf 10 °C gekühlt. Zu dieser Mischung wurde im Verlauf von 3 h portionsweise eine Mischung von 0,13 g (1,2 mmol, 0,9 mol%) N-Methylanilin und 0,14 g (1,2 mmol, 0,9 mol%) Trifluoressigsäure gegeben, und die Temperatur nach 20% der Zugabe auf 22 °C erhöht. Nach 3 h wurde das Wertprodukt aus der Reaktionsmischung durch fraktionierte Destillation im Vakuum isoliert. Siedepunkt 44-48 °C bei 17-18 mbar. Es wurden 10,0 g 5,5-Dimethyl-2-Isoxazolin erhalten, gemäß ¹H-NMR 89%ig (66%).
¹H-NMR (CDCl₃): 1,40 (s, 6H), 2,75 (d, 2H), 7,06 (br s, 1H).

### Beispiel 2: (Säure-Base Katalyse)

### Synthese von 5,5-Dimethyl-2-isoxazolin

50,0 g (0,684 mol, 100 mol%) Acetonoxim wurden mit 62,6 g (0,744 mol, 109 mol%) 3-Methyl-2-butenal versetzt und auf 10-15 °C gekühlt. Zu dieser Mischung wurden im Verlauf von 48 h 1,5 g (6,8 mmol, 1 mol%) N-Methylanilinium-Trifluoracetat in 0,1 g Portionen gegeben. Das Wertprodukt wurde anschließend aus der Reaktionsmischung durch fraktionierte Destillation im Vakuum isoliert. Siedepunkt 44-48 °C bei 17-18 mbar. Es wurden 56,9 g 5,5-Dimethyl-2-isoxazolin erhalten, gemäß ¹H-NMR >91%ig (76%).
¹H-NMR (CDCl₃): 1,40 (s, 6H), 2,75 (d, 2H), 7,06 (br s, 1 H).

### Beispiel 3: (Säure-Katalyse)

### Synthese von 5,5-Dimethyl-2-isoxazolin

4,35 g Acetonoxim (59,5 mmol, 100 mol%) und 5,27 g 3-Methyl-2-butenal (62,6 mmol, 105 mol%) wurden gemischt und mit 0.13 g Trifluoressigsäure (1,1 mmol, 1,9 mol%) versetzt. Es wurde 60 h bei Raumtemperatur gerührt und das Produkt im Vakuum destilliert (46°C, 18 mbar). Es wurden 4,7 g farbloses Öl erhalten, laut NMR mit einer Reinheit >95% (45,0 mmol, 76%).

### Beispiel 4: (Säure-Base Katalyse)

### Synthese von 5,5-Dimethyl-2-isoxazolin

0,4 g (2S,5S)-2-tert-Butyl-3-methyl-5-benzyl-4-imidazolinon (1,6 mmol, 1 mol%) wurde in 50 ml n-Pentan vorgelegt und bei 0 °C mit 0,19 g Trifluoressigsäure (1,6 mmol, 1 mol%) versetzt. Es wurde 30 min bei -3 - 0°C gerührt. Zu der entstandenen Suspension wurden bei 0 °C 11,9 g Acetonoxim (0,163 mol, 100 mol%) gegeben, die Mischung wurde auf 20 °C erwärmt, und 16,5 g 3-Methyl-2-butenal (0,196 mol, 120 mol%) innerhalb von 5 min zugetropft. Es wurde 16 h bei dieser Temperatur gerührt, und das Produkt durch Destillation isoliert. Es wurden 15,5 g 5,5-Dimethyl-2-isoxazolin mit einer Reinheit (GC) von 88%, entsprechend einer Ausbeute von 84% erhalten.

## Patentansprüche

1. Verfahren zur Herstellung 5,5-disubstituierter 2-Isoxazoline der Formel (I), 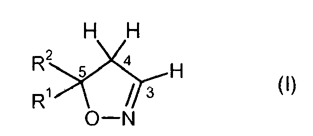 wobei
R¹, R² unabhängig voneinander jeweils C₁-C₆-Alkyl oder C₁-C₄-Halogenalkyl bedeuten; oder R¹ und R² zusammen eine C₂-C₅-Alkandiyl-Kette bilden, die ein- bis vierfach durch C₁-C₄-Alkyl substituiert sein kann und/oder durch Sauerstoff oder durch gegebenenfalls C₁-C₄-Alkyl-substituierten Stickstoff unterbrochen sein kann;
**dadurch gekennzeichnet, dass** ein Oxim der Formel (II) 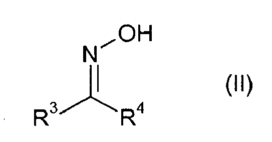 wobei
R³, R⁴ unabhängig voneinander jeweils Wasserstoff, C₁-C₆-Alkyl, C₁-C₄-Alkylcarbonyl, Hydroxyimino-C₁-C₄-alkyl, Phenyl, Phenyl-C₁-C₄-Alkyl oder Phenyl-C₂-C₄-Alkenyl bedeuten, wobei die Phenylringe ein- oder mehrfach durch C₁-C₄-Alkyl, C₁-C₄-Alkoxy, Di-C₁-C₄-Alkylamino, Halogen, Hydroxy oder Nitro substituiert sein können; oder R³ und R⁴ zusammen eine C₂-C₅-Alkandiyl-Kette bilden;
mit einer Carbonylverbindung der Formel (III), 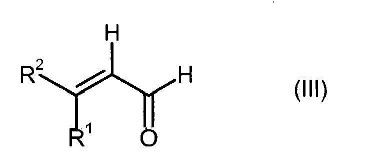 wobei R¹ und R² die oben genannten Bedeutungen haben;
in Anwesenheit eines Säurekatalysators oder eines Säure-Base-Katalysators und gegebenenfalls in Anwesenheit eines organischen Lösungsmittels umgesetzt wird.

2. Verfahren nach Anspruch 1, wobei
R¹ C₁-C₆-Alkyl oder C₁-C₄-Halogenalkyl;
R² C₁-C₆-Alkyl oder C₁-C₄-Halogenalkyl;
R³ Wasserstoff, C₁-C₆-Alkyl, C₁-C₄-Alkylcarbonyl, Hydroxyimino-C₁-C₄-alkyl; und
R⁴ C₁-C₆-Alkyl; bedeuten oder R³ und R⁴ eine C₂-C₅-Alkandiyl-Kette bilden.

3. Verfahren nach Anspruch 1 oder 2, wobei R¹ und R² jeweils Methyl bedeuten.

4. Verfahren nach einem der Ansprüche 1 bis 3, wobei R³ und R⁴ jeweils unabhängig voneinander Methyl oder Ethyl bedeuten.

5. Verfahren nach einem der Ansprüche 1 bis 4, **dadurch gekennzeichnet, dass** die Umsetzung der Verbindungen der Formel (II) und (III) mit weniger als 80 Gew% organischen Lösungsmittels im Reaktionsansatz gestartet wird.

6. Verfahren nach einem der Ansprüche 1 bis 4, **dadurch gekennzeichnet, dass** die Umsetzung der Verbindungen der Formel (II) und (III) ohne Zugabe eines organischen Lösungsmittels im Reaktionsansatz gestartet wird.

7. Verfahren nach einem der Ansprüche 1 bis 6, **dadurch gekennzeichnet, dass** als Katalysator ein Säurekatalysator eingesetzt wird.

8. Verfahren nach einem der Ansprüche 1 bis 6, **dadurch gekennzeichnet, dass** als Katalysator ein Säure-Base-Katalysator eingesetzt wird.

9. Verfahren nach einem der Ansprüche 1 bis 8, **dadurch gekennzeichnet, dass** das Molverhältnis der Verbindung der Formel (II) zum Katalysator unter 1:0.1 liegt.

10. Verfahren nach einem der Ansprüche 1 bis 8, **dadurch gekennzeichnet, dass** das Molverhältnis der Verbindung der Formel (II) zum Katalysator unter 1: 0.02 liegt.

## Claims

1. A process for preparing 5,5-disubstituted 2-isoxazolines of the formula (I) 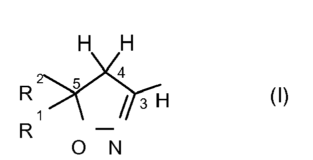 where
R¹, R² are each independently C₁-C₆-alkyl or C₁-C₄-haloalkyl; or R¹ and R² together form a C₂-C₅-alkanediyl chain which may be mono- to tetrasubstituted by C₁-C₄-alkyl and/or interrupted by oxygen or by optionally C₁-C₄-alkyl-substituted nitrogen;
which comprises reacting an oxime of the formula (II) 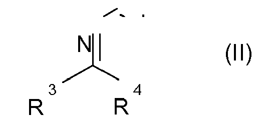 where
R³, R⁴ are each independently hydrogen, C₁-C₆-alkyl, C₁-C₄-alkylcarbonyl, hydroxyimino-C₁-C₄-alkyl, phenyl, phenyl-C₁-C₄-alkyl or phenyl-C₂-C₄-alkenyl, where the phenyl rings may be mono- or polysubstituted by C₁-C₄-alkyl, C₁-C₄-alkoxy, di-C₁-C₄-alkylamino, halogen, hydroxyl or nitro; or R³ and R⁴ together form a C₂-C₅-alkanediyl chain;
with a carbonyl compound of the formula (III) 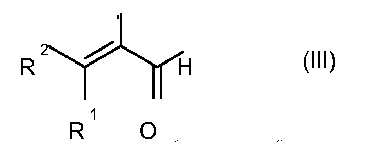 where R¹ and R² are each as defined above;
in the presence of an acid catalyst or an acid-base catalyst and optionally in the presence of an organic solvent.

2. The process according to claim 1, wherein
R¹ is C₁-C₆-alkyl or C₁-C₄-haloalkyl;
R² is C₁-C₆-alkyl or C₁-C₄-haloalkyl;
R³ is hydrogen, C₁-C₆-alkyl, C₁-C₄-alkylcarbonyl, hydroxyimino-C₁-C₄-alkyl; and
R⁴ is C₁-C₆-alkyl; or R³ and R⁴ form a C₂-C₅-alkanediyl chain.

3. The process according to claim 1 or 2, wherein R¹ and R² are each methyl.

4. The process according to any one of claims 1 to 3, wherein R³ and R⁴ are each independently methyl or ethyl.

5. The process according to any one of claims 1 to 4, wherein the reaction of the compounds of the formulae (II) and (III) is started with less then 80% by weight of organic solvent in the reaction mixture.

6. The process according to any one of claims 1 to 4, wherein the reaction of the compounds of the formulae (II) and (III) is started without addition of an organic solvent in the reaction mixture.

7. The process according to any one of claims 1 to 6, wherein the catalyst used is an acid catalyst.

8. The process according to any one of claims 1 to 6, wherein the catalyst used is an acid-base catalyst.

9. The process according to any one of claims 1 to 8, wherein the molar ratio of the compound of the formula (II) to the catalyst is less than 1:0.1.

10. The process according to any one of claims 1 to 8, wherein the molar ratio of the compound of the formula (II) to the catalyst is less than 1:0.02.

## Revendications

1. Procédé pour la préparation de 2-isoxazolines disubstituées en 5ème position de formule (I) 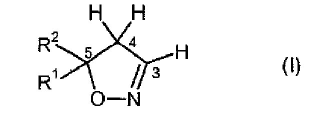 où
R¹, R² signifient, indépendamment l'un de l'autre, à chaque fois C₁-C₆-alkyle ou C₁-C₄-halogénoalkyle ; ou R¹ et R² forment ensemble une chaîne C₂-C₅-alcanediyle qui peut être monosubstituée à tétrasubstituée par C₁-C₄-alkyle et/ou être interrompue par de l'oxygène ou par de l'azote le cas échéant substitué par C₁-C₄-alkyle ; caractérisé
en ce d'une oxime de formule (II) 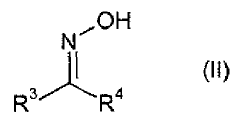 où
R³, R⁴ signifient, indépendamment l'un de l'autre, à chaque fois hydrogène, C₁-C₆-alkyle, C₁-C₄-alkylcarbonyle, hydroxymino-C₁-C₄-alkyle, phényle, phényl-C₁₋C₄-alkyle ou phényl-C₂-C₄-alcényle, les cycles phényle pouvant être monosubstitués ou polysubstitués par C₁-C₄-alkyle, C₁-C₄-alcoxy, di-C₁-C₄-alkylamino, halogène, hydroxy ou nitro ; ou R³ et R⁴ pouvant former ensemble une chaîne C₂-C₅-alcanediyle ;
est transformée avec un composé carbonyle de formule (III) 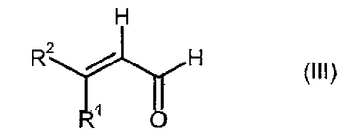 R¹ et R² ayant les significations susmentionnées ;
en présence d'un catalyseur acide ou d'un catalyseur acide-base et le cas échéant en présence d'un solvant organique.

2. Procédé selon la revendication 1,
R¹ signifiant C₁-C₆-alkyle ou C₁-C₄-halogénoalkyle ;
R² signifiant C₁-C₆-alkyle ou C₁-C₄-halogénoalkyle ;
R³ signifiant hydrogène, C₁-C₆-alkyle, C₁-C₄-alkylcarbonyle, hydroxyimino-C₁-C₄-alkyle ; et
R⁴ signifiant C₁-C₆-alkyle ;
ou R³ et R⁴ formant une chaîne C₂-C₅-alcanediyle.

3. Procédé selon la revendication 1 ou 2, R¹ et R² signifiant à chaque fois méthyle.

4. Procédé selon l'une quelconque des revendications 1 à 3, R³ et R⁴ signifiant à chaque fois, indépendamment l'un de l'autre, méthyle ou éthyle.

5. Procédé selon l'une quelconque des revendications 1 à 4, **caractérisé en ce que** la transformation des composés de formule (II) et (III) est initiée avec moins de 80% en poids de solvant organique dans la charge réactionnelle.

6. Procédé selon l'une quelconque des revendications 1 à 4, **caractérisé en ce que** la transformation des composés de formule (II) et (III) est initiée sans addition de solvant organique dans la charge réactionnelle.

7. Procédé selon l'une quelconque des revendications 1 à 6, **caractérisé en ce qu'**on utilise, comme catalyseur, un catalyseur acide.

8. Procédé selon l'une quelconque des revendications 1 à 6, **caractérisé en ce qu'**on utilise, comme catalyseur, un catalyseur acide-base.

9. Procédé selon l'une quelconque des revendications 1 à 8, **caractérisé en ce que** le rapport molaire du composé de formule (II) au catalyseur est inférieur à 1:0,1.

10. Procédé selon l'une quelconque des revendications 1 à 8, **caractérisé en ce que** le rapport molaire du composé de formule (II) au catalyseur est inférieur à 1:0,02.
